(19) Europäisches Patentamt
European Patent Office
Office européen des brevets

(11) **EP 3 481 548 B1**

(12) **FASCICULE DE BREVET EUROPEEN**

(45) Date de publication et mention
de la délivrance du brevet:
**24.06.2020 Bulletin 2020/26**

(21) Numéro de dépôt: **17746170.4**

(22) Date de dépôt: **07.07.2017**

(51) Int Cl.:
*B01J 20/10* (2006.01)     *B01J 20/22* (2006.01)
*B01J 20/28* (2006.01)     *B01D 53/02* (2006.01)
*B01D 53/66* (2006.01)     *G01N 31/22* (2006.01)
*G01N 21/78* (2006.01)

(86) Numéro de dépôt international:
**PCT/FR2017/051868**

(87) Numéro de publication internationale:
**WO 2018/007772 (11.01.2018 Gazette 2018/02)**

(54) **MATRICE NANOPOREUSE ET SON UTILISATION**

NANOPORÖSE MATRIX UND VERWENDUNG DAVON

NANOPOROUS MATRIX AND USE THEREOF

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **08.07.2016 FR 1656600**

(43) Date de publication de la demande:
**15.05.2019 Bulletin 2019/20**

(73) Titulaires:
• **Commissariat à l'Énergie Atomique
et aux Énergies Alternatives
75015 Paris (FR)**
• **Centre National de la Recherche Scientifique
75016 Paris (FR)**

(72) Inventeurs:
• **TRAN-THI, Thu-Hoa
92120 Montrouge (FR)**
• **CAILLAT, Ludovic
94310 Orly (FR)**

(74) Mandataire: **Plasseraud IP
66, rue de la Chaussée d'Antin
75440 Paris Cedex 09 (FR)**

(56) Documents cités:
**EP-A1- 3 015 165         EP-A2- 1 394 541
WO-A1-2008/056513    DE-U1- 9 217 354
JP-A- 2008 086 950**

**Description**

L'invention concerne un procédé de préparation de nouvelles matrices nanoporeuses, les matrices nanoporeuses ainsi obtenues ainsi que leur utilisation, notamment comme filtre d'abattement d'ozone et capteur d'ozone.

Les effets préoccupants de la pollution atmosphérique sur la santé ont conduit les pouvoirs publics à mettre en place des normes de plus en plus restrictives sur les émissions des polluants. Parmi l'ensemble des polluants, l'ozone représente un problème majeur quand sa concentration dans l'atmosphère dépasse le seuil d'alerte de 75 ppb (180 $\mu$g.m$^{-3}$) sur 1 heure.

Or dans certains espaces confinés tels que l'habitacle de voiture, des avions [1] ou les centres de photocopie équipés de photocopieuses et d'imprimantes-laser [2], la concentration d'ozone peut atteindre des valeurs beaucoup plus élevées, largement supérieures à 100 ppb. L'ozone est extrêmement nocif pour les poumons, les reins, le cerveau et les yeux ; il est notamment à l'origine de problèmes respiratoires tels que des crises d'asthme chez les sujets sensibles. Selon l'OMS, il représente des problèmes lorsque sa concentration dans l'atmosphère augmente au-delà du seuil de 50 ppb (soit 120 $\mu$g.m$^{-3}$) par jour pendant 8h.

L'éradication de ce polluant nocif dans l'air intérieur et notamment dans les espaces publics ou de travail confinés ainsi que la surveillance de l'ozone en temps réel est un enjeu important pour la protection de la population.

Les filtres les plus communément utilisés pour piéger l'ozone sont des filtres moléculaires à base de charbon actif ou d'alumine imprégnée de permanganate de potassium ($KMnO_4$). Les filtres moléculaires dits rigides sont composés de charbon actif (1,25 kg/filtre) protégés en amont par des préfiltres plissés (médium fibres de verre, société CAMFIL). Les charbons actifs en grain (diamètre > 2,5 mm) sont fabriqués à partir des noix de coco et présentent une surface spécifique de 800 m$^2$.g$^{-1}$. Ces filtres ont une efficacité vis-à-vis d'$O_3$ de l'ordre de 58 à 79% et peuvent avoir des durées de vie de plusieurs années avec cependant une efficacité qui baisse graduellement avec le temps dès les premiers 6 mois [3].

Malgré de bons rendements et une bonne durabilité, les charbons actifs possèdent des inconvénients. Pour des humidités relatives importantes de l'ordre de 70%, l'efficacité de piégeage diminue fortement du fait de l'adsorption compétitive entre les molécules d'eau sous forme de vapeur et l'ozone. Par ailleurs, il n'existe pas de moyen de contrôle de la saturation des charbons actifs, ces derniers étant connus pour relarguer les polluants dès leur saturation.

Un autre filtre d'ozone connu est l'iodure de potassium (KI) qui réagit avec l'$O_3$ pour produire soit du $I_2$ et $I_3^-$ en milieu humide, soit du $KIO_3$ en milieu sec. Cependant, le KI réagit avec tous les oxydants et un rejet de $I_2$ dans l'air ambiant n'est pas recommandé.

D'autres systèmes catalytiques sont également utilisés pour la destruction de l'ozone. De nombreux métaux précieux et d'oxydes métalliques ont été utilisés à cet effet [4]. Ces derniers moins coûteux que les métaux précieux ont fait l'objet de nombreuses études. Une comparaison de l'efficacité des oxydes métalliques donne : $MnO_2$ (42%)> $Co_3O_4$ (39%)> NiO (35%)> $Fe_2O_3$ (24%)> $Ag_2O$ (21%)> $Cr_2O_3$ (18%)> $CeO_2$ (11%)> MgO (8%)> $V_2O_5$ (8%)> CuO (5%)> $MoO_3$ (4%) [4]. Ces systèmes catalytiques doivent néanmoins fonctionner à haute température (200 à 400 °C) pour une bonne efficacité et sont difficilement utilisables en continu pendant des mois voire des années. De ce fait, ils sont plutôt utilisés dans certains avions avec un fonctionnement qui ne dure que la durée du voyage. Par ailleurs, les systèmes catalytiques ne sont pas spécifiques à l'ozone.

A l'heure actuelle, parmi tous ces filtres, les charbons actifs restent les meilleurs candidats en termes de rapport qualité/prix et de durabilité. Toutefois, les charbons actifs ne piègent pas uniquement l'$O_3$. Ils piègent toutes sortes de composés volatils présents dans l'atmosphère et il est impossible de connaître l'état de saturation d'un charbon actif et le début de relargage des composés nocifs.

Récemment, de l'indigo, sous forme de poudre ou de film mince déposé sur des capteurs à base de semiconducteurs, a été utilisé comme filtre spécifique pour discriminer $O_3$ et $NO_2$ [5]. L'indigo a souvent été utilisé pour la réalisation de capteurs chimiques d'$O_3$ [6,7]. Dans ces derniers cas, on le retrouve soit dissous dans un polymère [6], soit dans des matrices poreuses par imprégnation en solution [7] pour la détection de faibles teneurs d'$O_3$. Dans tous les cas cités, sous forme de poudre, de film mince, ou de matrices poreuses dopées, les surfaces spécifiques d'adsorption sont trop faibles pour une application de filtre. EP 1 394 541 A2 divulgue une matrice poreuse sol-gel renfermant un colorant comme par exemple l'indigo carmine pour le piégeage et la détection de l'ozone.

Une imprégnation des charbons actifs avec de l'indigo n'est pas envisageable, car l'efficacité des charbons actifs réside en leurs tailles de pore très petites. Or les tailles de pore trop petites ne permettent pas de séquestrer les molécules d'indigo. Une couverture des surfaces externes des grains de charbon avec l'indigo n'apporterait qu'une faible surface d'adsorption et par conséquence une efficacité moindre et non durable.

Par ailleurs, une imprégnation des charbons actifs avec de l'iodure de potassium, qui pourrait entrer dans les pores du charbon actif, pourrait avoir pour effet un relargage d'$I_2$ lorsque le charbon actif est saturé. De plus, dans ce cas, il n'est toujours pas possible de connaître l'état de saturation du filtre.

La présente invention a pour but de remédier aux problèmes rencontrés avec les filtres d'$O_3$ ou des systèmes d'abattement de polluants commercialisés ou décrits dans la littérature. Par rapport au charbon actif, ces problèmes sont notamment le manque de spécificité, l'absence d'alerte à la saturation, le relargage des polluants à la saturation et l'interférence avec l'humidité de l'air. Dans le cas de la poudre d'iodure de potassium, il s'agit notamment du manque de surface spécifique d'absorption et du relargage de composés toxiques ($I_2$). Le manque de surface spécifique est également rencontré avec les matrices poreuses imprégnées d'indigo en solution. Enfin, les systèmes catalytiques nécessitent un fonctionnement à haute température pour un abattement non spécifique des polluants.

La présente invention a également pour but de proposer une méthode simple et facilement industrialisable de production de filtres spécifiques à l'ozone dotés d'une fonction alerte de saturation.

Outre le piégeage de l'ozone avec des filtres, la mesure de la concentration de l'ozone dans l'air est un enjeu important pour la protection de la population. En matière de mesures, plusieurs principes de transduction tels que la spectrométrie de masse, la chromatographie ou encore la spectrophotométrie d'absorption Ultraviolet sont aujourd'hui exploités commercialement pour détecter l'ozone. Les analyseurs d'ozone généralement utilisés dans les stations de mesure des associations agréées de surveillance pour la qualité de l'air (AASQA) reposent sur la spectrophotométrie d'absorption Ultraviolet. Cette dernière méthode est basée sur la mesure directe de l'absorbance de l'ozone à 254 nm. Ces systèmes sont très performants, mais aussi très volumineux et très coûteux ce qui réduit leur utilisation pour des mesures sur sites où la portabilité des appareillages est requise.

La mesure dite en « temps réel » a donc suscité de nombreux développements de capteurs basés sur différents principes [8]. Pour la mesure de l'ozone dans l'atmosphère en air extérieur ou intérieur, nous ne nous intéresserons qu'aux capteurs colorimétriques à coût modique, fonctionnant soit en mode actif ou passif et capables de mesurer des niveaux ppb d'$O_3$.

Dans le brevet US 4,859,607 du 22 Août 1989 [9], Lambert et al. ont proposé un capteur colorimétrique à base d'une azine de la 3-méthyl-2-benzothiazoline acétone mélangé en proportion 1:4 avec du 2-phénylphénol. Le mélange dissous dans l'acétone est déposé sur un substrat solide. En présence d'ozone, le mélange devient rouge violacé. Bien que l'intensité de la coloration ne varie pas linéairement avec la concentration d'ozone, ces auteurs proposent de l'utiliser comme un détecteur passif d'ozone pour des teneurs d'ozone de 100 ppb au ppm. Ce capteur colorimétrique est sélectif à l'ozone et ne réagit pas avec $O_2$, $NO_2$, $SO_2$, $Br_2$, $I_2$ mais donne une coloration jaune avec $Cl_2$.

Le colorant le plus populaire de la littérature demeure l'indigo, initialement utilisé pour colorer les tissus. En 2004, Franklin et al. ont réalisé des capteurs d'ozone en utilisant du papier de cellulose imprégné d'indigo carmine pour la mesure d'ozone dans l'air extérieur [10]. Le papier de cellulose étant opaque, ces auteurs ont mesuré le changement de couleur par réflectance et trouvent que la méthode est moins fiable que les capteurs passifs à base de nitrite. Ils attribuent ce résultat à l'existence d'interférences dans l'air extérieur sans cependant identifier leur nature.

En 2005, Marcy et al. ont proposé l'utilisation de divers colorants dérivés de l'indigo pour la mesure sélective de l'ozone [11]. Ces auteurs ont dissous ces composés dans un copolymère de polydiméthylsiloxane et de polycarbonate et déposé ce mélange sur un substrat solide transparent. Le film de ~580 nm d'épaisseur est perméable à $O_3$ grâce à la présence du polydiméthylsiloxane. L'analyse du déclin de l'absorbance de l'indigo à 663 nm en fonction du temps et de la concentration d'$O_3$ et en présence de divers interférents potentiels, $NO_2$ et $Cl_2$, ont permis à ces auteurs de conclure à une bonne sélectivité du capteur. La réponse du capteur à $O_3$ en mode dynamique de détection (1L.min$^{-1}$ et Humidité relative <4%) est de 0,1118 ppm$^{-1}$.s$^{-1}$ et la limite de détection, de 30 ppb reste encore élevée pour la mesure de la qualité de l'air.

En 2007, la compagnie japonaise NTT propose un capteur colorimétrique d'ozone qui combine l'indigo carmine et un matériau poreux servant de support, le verre Vycor 7930. Ce verre présente des pores de 6 nm de diamètre et possède une surface spécifique d'adsorption de 250 m$^2$.g$^{-1}$ [12,13]. Les auteurs rapportent que l'un des problèmes dans la préparation du capteur est l'imprégnation du substrat. En effet, l'indigo carmine ne rentre dans les pores du verre Vycor que lorsque la solution d'imprégnation est à pH acide < 5. Or à pH acide, l'indigo carmine protoné n'est pas stable en solution. Cependant, ces auteurs mentionnent que l'indigo carmine peut demeurer stable (au moins 6 mois) dans les pores du Vycor, une fois le solvant évaporé. Avec ce capteur fonctionnant en mode passif, les auteurs donnent une limite de détection de 3 ppb pour 1H d'exposition. NTT a néanmoins identifié une interférence majeure de ce capteur qui a une sensibilité aux radiations UV gênante pour son utilisation en milieu extérieur.

Pour pallier à ce problème de sensibilité aux rayonnements UV, NTT propose d'incorporer un composé « absorbeur d'UV » et d'utiliser du papier comme substrat pour la réalisation du capteur d'ozone [14]. Le nouveau capteur proposé est un papier trempé dans un premier temps dans une solution acidifiée (acide citrique) d'indigo carmine contenant un épaississant (glycérine), puis trempé une 2^ème fois dans une solution d'acétone contenant un absorbeur d'UV (2-hydroxy-4-methoxybenzophenone-5-sulfonic acid, ou 2-hydroxy-4-methoxybenzophenone-5-sodium sulfonate, ou ferulic acid (4-hydroxy-3-methoxy cinnamic acid)), puis séché pour l'usage considéré.

Cependant, il est connu de la littérature que les dérivés du benzophénone, souvent utilisés comme absorbeurs de rayonnement UV, réagissent eux-mêmes avec l'ozone [15]. Parmi ceux-ci, le 2,2'-dihydroxy-4-methoxybenzophenone est le plus réactif vis-à-vis de l'ozone et produit de nombreux produits de dégradation. Ainsi en présence de dérivé de benzophénone, les teneurs d'ozone mesurées pourraient être sous-évaluées. De même, la réactivité de l'ozone vis-à-vis des composés aromatiques substitués est également connue [16]. Ainsi, l'emploi de l'acide férulique pourrait éga-

3

lement contribuer à fausser la mesure de l'ozone.

La présente invention a également pour but de remédier aux problèmes rencontrés avec les capteurs d'O$_3$ à base de dérivés d'indigo commercialisés ou décrits dans la littérature. La présente invention vise à pallier tout ou partie des inconvénients identifiés ci-dessus, notamment en évitant la méthode de dopage par imprégnation.

Il est du mérite des inventeurs d'avoir découvert, de manière très inattendue et après de nombreuses recherches, qu'il était possible de préparer des matrices sol-gel nanoporeuses dont la distribution de tailles de pores est choisie pour à la fois laisser passer l'ozone et séquestrer la molécule sonde, conférant à la matrice à la fois une fonction de filtration de l'air pollué à l'ozone avec une fonction alerte à la saturation du filtre et une possibilité de suivi quantitatif de l'ozone présent dans l'atmosphère.

Une matrice sol gel est un matériau obtenu par un procédé sol-gel consistant à utiliser comme précurseurs des alcoxydes métalliques de formule M(OR)$_x$R'$_{n-x}$ ou M est un métal, notamment le silicium, R un groupement alkyle et R' un groupement porteur d'une ou de plusieurs fonctions avec n= 4 et x pouvant varier entre 2 et 4. En présence d'eau, les groupements alkoxy (OR) sont hydrolysés en groupements silanols (Si-OH). Ces derniers se condensent en formant des liaisons siloxane (Si-O-Si-). Il se forme des petites particules de taille généralement inférieure à 1 $\mu$m, qui s'agrègent et forment des amas qui restent en suspension sans précipiter, formant un sol. L'augmentation des amas et leur condensation augmente la viscosité du milieu qui gélifie. Un matériau solide poreux est obtenu par séchage du gel, avec l'expulsion du solvant en dehors du réseau polymérique formé (synérèse).

Un objet de l'invention se rapporte donc à un procédé de préparation d'une matrice sol-gel polyalcoxysilane nanoporeuse renfermant de l'indigo carmine, ledit procédé comprenant les étapes suivantes :

a) synthèse d'un gel à partir de tétraméthoxysilane ou d'un mélange de tétraméthoxysilane et d'un autre précurseur organosilicé choisi parmi le phényltriméthoxysilane, le phényltriéthoxysilane, un fluoroalkyltriméthoxysilane, un fluoroalkyltriéthoxysilane, un chloroalkylméthoxysilane, un chloroalkyléthoxysilane, un aminopropyltriéthoxysilane et leurs mélanges, la synthèse étant effectuée en milieu aqueux en présence d'un solvant organique polaire et de l'indigo carmine à une température allant de 20 à 70 °C

b) séchage du gel obtenu à l'étape a) de façon à obtenir une matrice sol-gel nanoporeuse renfermant de l'indigo carminé.

Les matrices sol-gel polyalcoxysilane nanoporeuses ainsi obtenues ont une proportion de micropores supérieure ou égale à 10 %, de préférence supérieure ou égale à 20 %, 30%, 40%, 50 %, 60% ou 80%. Ces micropores empêchent les molécules d'indigo carmine de sortir. L'ozone, quant à lui, peut facilement diffuser dans le réseau poreux à l'inverse des molécules de grande taille comme les composés aromatiques monocycliques. Lorsque l'ozone entre en contact avec l'indigo carmine, il réagit avec celui-ci par addition électrophile à la liaison C-C insaturée pour donner de l'isatine ou de l'anhydride isatoique comme principaux produits de réaction. Cette réaction est accompagnée d'un changement de couleur, l'indigo carmine étant de couler bleu foncé et les produits de réaction de couleur jaune. Ainsi, la matrice nanoporeuse selon l'invention change progressivement de couleur du bleu foncé via le vert jusqu'au jaune lorsqu'elle est exposé à de l'ozone, par exemple présent dans l'air ambiant intérieur ou extérieur.

On entend ici par micropores des pores dont le diamètre est inférieur à 2 nm. En général le diamètre de micropores est de 0,8 à 2 nm. Dans la présente invention, le diamètre des micropores est avantageusement centré autour de 1,1$\pm$0,1 nm, c'est-à-dire au moins 40 à 60 % des micropores ont un diamètre d'environ 1,1$\pm$0,1 nm.

Les matrices sol-gel nanoporeuses selon l'invention présentent avantageusement une proportion de micropores de 10% à 100%, de préférence de 20% à 90%, plus préférentiellement de 30% à 80% et une proportion de mésopores de 0% à 90%, de préférence de 10% à 80%, plus préférentiellement de 20% à 70%. Plus préférentiellement encore, elles présentent une proportion de micropores de 50 à 70% et une proportion de mésopores de 50 à 30%.

L'homme du métier saura choisir les proportions *ad hoc* de micropores / mésopores en fonction de l'application visée et de ses contraintes. Ainsi, pour la fonction filtre, l'objectif est de piéger le plus possible d'O$_3$, tout en assurant une durée de vie élevée du filtre et en évitant tout relargage. On privilégiera donc les proportions de micropores élevées et préférentiellement supérieures à 50% et plus préférentiellement supérieures à 80%. Pour la fonction capteur, dont le principe est le changement de couleur (qui est proportionnel à la teneur d'ozone), l'objectif principal est de mesurer rapidement la teneur d'ozone dans l'atmosphère. Pour ce faire, il faut permettre une diffusion rapide de l'ozone dans la matrice afin d'obtenir un changement de coloration rapide. On privilégiera alors les proportions de mésopores élevées et préférentiellement supérieures à 30% et plus préférentiellement supérieures à 60%. Avantageusement, on ne dépassera pas une proportion de micropores de 70% pour la fonction capteur. Les matrices sol-gel nanoporeuses selon l'invention présentent également une surface spécifique de 550$\pm$50 m$^2$.g$^{-1}$ à 890$\pm$80 m$^2$.g$^{-1}$ de préférence de 650$\pm$70. m$^2$.g$^{-1}$ à 890$\pm$80 m$^2$.g$^{-1}$ et plus préférentiellement encore de 750$\pm$70 m$^2$.g$^{-1}$ à 890$\pm$80 m$^2$.g$^{-1}$.

La surface spécifique et la distribution des tailles de pores sont déterminées par analyse de l'isotherme d'adsorption-désorption à l'azote liquide avec le modèle DFT (Théorie de la Fonctionnelle de Densité).

La synthèse du gel à l'étape a) du procédé selon l'invention est avantageusement une synthèse monotope (anglais : one-pot), c'est-à-dire elle est réalisée en une seule étape avec le tétraméthoxysilane ou le mélange de tétraméthoxysilane et l'autre précurseur organosilicé, et l'indigo carmine en présence du solvant polaire et l'eau.

La synthèse du gel à l'étape a) est avantageusement réalisée à partir de tétraméthoxysilane ou d'un mélange de tétraméthoxysilane et d'un autre précurseur organosilicé choisi parmi le phényltriméthoxysilane (PhTMOS), le phényltriéthoxysilane (PhTEOS), un fluoroalkyltriméthoxysilane, un chloroalkylméthoxysilane, un aminopropyltriéthoxysilane et leurs mélanges, de préférence parmi le phényltriémthoxysilane (PhTMOS), le phényltriéthoxysilane (PhTEOS), un fluoropropyltriméthoxysilane, un chloropropylméthoxysilane, un aminopropyltriéthoxysilane et leurs mélanges.

Dans un mode de réalisation, la synthèse du gel est réalisée à partir de tétraméthoxysilane. Dans un autre mode de réalisation, la synthèse du gel est réalisée à partir d'un mélange de tétraméthoxysilane et d'un autre précurseur organosilicé choisi parmi le phényltriméthoxysilane, le phényltriéthoxysilane, un fluoroalkyltriméthoxysilane, un fluoroalkyltriéthoxysilane, un chloroalkyltriméthoxysilane, un chloroalkyltriéthoxysilane, un aminopropyltriéthoxysilane et leurs mélanges, de préférence parmi un fluoropropyltriméthoxysilane, un fluoropropyltriéthoxysilane, un chloropropyltriméthoxysilane, un chloropropyltriéthoxysilane, un aminopropyltriéthoxysilane et leurs mélanges, de préférence parmi le phényltriméthoxysilane, le phényltriéthoxysilane, un fluoropropyltriméthoxysilane, un chloropropyltriméthoxysilane et un aminopropyltriéthoxysilane, de préférence encore parmi le phényltriéméthoxysilane, le phényltriéthoxysilane, le triméthoxy(3,3,3-trifluoropropyl)silane (3FTMOS), le (3-chloropropyl)triméthoxysilane (3ClTMOS) et le (3-aminopropyl)triéthoxysilane.

Lors de l'utilisation d'un mélange de tétraméthoxysilane et d'un autre précurseur organosilicé les proportions molaires de tétraméthoxysilane/autre précurseur organosilicé peuvent être variées entre 0,97/0,03 et 0,6/0,4, de préférence entre 0,9/0,1 et 0,7/0,3.

En effet, les matrices sol-gel à base de tétraméthoxysilane absorbent dans l'UV entre 200 et 350 nm ce qui leur confère un effet protecteur aux radiations UV nécessaire pour la stabilité de l'indigo carmine. Cet effet est encore augmenté par l'ajout de phényltriméthoxysilane, de phényltriéthoxysilane, d'un fluoroalkyltriméthoxysilane, d'un chloroalkyltriméthoxysilane, d'un aminopropyltriméthoxysilane ou d'un de leurs mélanges. De plus, parmi les composés utilisés comme écrans aux radiations UV, les groupements phényle [7] sont les moins réactifs vis-à-vis de l'ozone.

L'apport de groupements phényle et de longues chaînes carbonées et fluorées augmente par ailleurs l'hydrophobicité de la matrice, ce qui réduit l'interférence à la vapeur d'eau. Ainsi, dans un mode de réalisation particulier, le gel de l'étape a) est préparé à partir d'un mélange de tétraméthoxysilane et d'un autre précurseur organosilicé choisi parmi le phényltriméthoxysilane, le phényltriéthoxysilane, un fluoroalkyltriméthoxysilane, un chloroalkyltriméthoxysilane et leurs mélanges, de préférence parmi le phényltriméthoxysilane, le phényltriéthoxysilane, et un fluoroalkyltriméthoxysilane, de préférence parmi le phényltriméthoxysilane, le phényltriéthoxysilane, et un fluoropropyltriméthoxysilane, de préférence encore parmi le phényltriméthoxysilane, le phényltriéthoxysilane et le triméthoxy(3,3,3-trifluoropropyl)silane.

L'apport de groupement phényle, de chloropropyltriméthoxysilane et de fluoroalkyltriméthoxysilane permet également d'augmenter le nombre de micropores (diamètre <20Å) par rapport aux mésopores (20<diamètre<50Å) et d'améliorer la distribution des micropores de façon à favoriser encore plus le piégeage sélectif de petites molécules comme l'ozone. Ainsi, dans un mode de réalisation particulièrement avantageux, le gel de l'étape a) est préparé à partir d'un mélange de tétraméthoxysilane et du phényltriméthoxysilane ou du phényltriéthoxysilane.

Le solvant organique polaire mis en œuvre lors de la synthèse du gel à l'étape a) est avantageusement un solvant organique protique, de préférence un alcool aliphatique en C1 à C6, plus préférentiellement du méthanol ou de l'éthanol et plus préférentiellement encore du méthanol. Les proportions molaires de solvant organique polaire et d'eau peuvent varier respectivement entre 4 et 10, de préférence entre 4 et 7 et plus préférentiellement entre 4 et 5.

La préparation du gel à l'étape a) est avantageusement effectuée avec $10^{-5}$ mol.L$^{-1}$ à $10^{-1}$ mol.L$^{-1}$, de préférence $2 \cdot 10^{-4}$ mol.L$^{-1}$ à $5 \cdot 10^{-2}$ mol.L$^{-1}$ et plus préférentiellement encore $5 \cdot 10^{-5}$ mol.L$^{-1}$ à $3 \cdot 10^{-2}$ mol.L$^{-1}$ d'indigo carmine. En dessous de $10^{-4}$ mol.L$^{-1}$, la matrice nanoporeuse ne contiendra pas assez d'indigo carmine pour piéger efficacement l'ozone pendant une longue durée et au-delà de $10^{-1}$ mol.L$^{-1}$, la limitation est la solubilité de l'indigo carmine. Avec ces concentrations de départ on obtient à l'issue de l'étape b) du procédé selon l'invention une matrice nanoporeuse ayant une teneur en indigo carmine de $10^{-4}$ mol.dm$^3$ à 1 mol.dm$^3$, de préférence de $2 \cdot 10^{-3}$ mol.dm$^3$ à $5 \cdot 10^{-1}$ mol.dm$^3$, plus préférentiellement encore de $5 \cdot 10^{-4}$ mol.dm$^3$ à $3 \cdot 10^{-1}$ mol.dm$^3$.

Dans un mode de réalisation, la préparation du gel à l'étape a) est avantageusement effectuée avec $10^{-4}$ à $10^{-1}$ mol.L$^{-1}$, de préférence $10^{-3}$ à $5 \cdot 10^{-2}$ mol.L$^{-1}$ et plus préférentiellement encore $10^{-2}$ à $3 \cdot 10^{-2}$ mol.L$^{-1}$ d'indigo carmine. Avec ces concentrations de départ on obtient à l'issue de l'étape b) du procédé selon l'invention une matrice nanoporeuse ayant une teneur en indigo carmine de $10^{-3}$ à 1 mol.dm$^3$, de préférence de $10^{-2}$ à $5 \cdot 10^{-1}$ mol.dm$^3$, plus préférentiellement encore de $10^{-1}$ mol.dm$^3$ à $3 \cdot 10^{-1}$ mol.dm$^3$.

Dans un autre mode de réalisation, la préparation du gel à l'étape a) est avantageusement effectuée avec $10^{-5}$ à $2 \cdot 10^{-4}$ mol.L$^{-1}$, de préférence $2 \cdot 10^{-5}$ à $1,5 \cdot 10^{-4}$ mol.L$^{-1}$ et plus préférentiellement encore $5 \cdot 10^{-5}$ à $10^{-4}$ mol.L$^{-1}$ d'indigo carmine. Avec ces concentrations de départ on obtient à l'issue de l'étape b) du procédé selon l'invention une matrice nanoporeuse ayant une teneur en indigo carmine de $10^{-4}$ à $2 \cdot 10^{-3}$ mol.dm$^3$, de préférence de $2 \cdot 10^{-4}$ à $1,5 \cdot 10^{-3}$ mol.dm$^3$, plus préférentiellement encore de $5 \cdot 10^{-4}$ à $10^{-3}$ mol.dm$^3$.

La préparation du gel à l'étape a) est avantageusement effectuée à une température de 20 à 70 °C. L'utilisation d'une température élevée permet d'accélérer l'hydrolyse et la condensation des précurseurs organosilicés. Par exemple un chauffage du mélange réactionnel (sol) à 60°C permet d'accélérer la gélification d'un facteur 6 à 7 par rapport à une préparation du gel à température ambiant (20-25°C).

La préparation du gel est avantageusement réalisée en mélangeant d'abord le ou les précurseurs organosilicés avec le solvant organique polaire suivi de l'ajout d'une solution aqueuse d'indigo carmine. Le mélange ainsi obtenu est mélangé pour obtenir un sol. Ce sol est ensuite versé dans un ou plusieurs moules et laissé reposer pour obtenir un gel. Ce gel est enfin séché par évaporation de l'eau et du solvant organique pour obtenir la matrice sol-gel nanoporeuse renfermant de l'indigo carmine selon l'invention. L'évaporation de l'eau et du solvant pour le séchage de la matrice peut être réalisé selon toute méthode connue de l'homme du métier. Le séchage peut par exemple être effectué à température ambiante ou à une température plus élevée, notamment de 40 à 80 °C. De préférence, le séchage est réalisé à une température allant de la température ambiante (environ 20°C) à 50°C. Avantageusement, le séchage est effectué sous atmosphère inerte.

L'invention concerne également la matrice sol-gel polyalcoxysilane nanoporeuse renfermant de l'indigo carmine susceptible d'être obtenu par le procédé conforme à l'invention en tant que telle. Une telle matrice est une matrice sol-gel polyalcoxysilane obtenue à partir de tétraméthoxysilane ou d'un mélange de tétraméthoxysilane et d'un autre précurseur organosilicé et est notamment caractérisée en ce qu'elle présente une surface spécifique comprise entre $650\pm60$ et $890\pm80$ $m^2.g^{-1}$, notamment entre $750\pm70$ et $890\pm80$ $m^2.g^{-1}$, et une proportion de micropores de supérieure à 30 %, de préférence supérieure à 40 % et plus préférentiellement encore supérieure à 60 %. Avantageusement, cette matrice a une teneur en indigo carmine de $10^{-3}$ à $10^{-2}$ $mol.dm^3$, de préférence de $10^{-2}$ à $5\ 10^{-2}$ $mol.dm^3$, plus préférentiellement encore de $10^{-1}$. $mol.dm^3$ à $3\ 10^{-1}$ $mol.dm^3$.

Dans un mode de réalisation, la matrice sol-gel selon l'invention présente une proportion de micropores de 30 à 100% et une proportion de mésopores de 70 à 0%, de préférence une proportion de micropores de 40 à 90% et une proportion de mésopores de 60 à 10%, plus préférentiellement une proportion de micropores de 50 à 70% et une proportion de mésopores de 50 à 30%. Avantageusement, la matrice de ce mode de réalisation présente une surface spécifique comprise entre $550\pm50$ et $890\pm80$ $m^2.g^{-1}$, notamment entre $650\pm70$ et $890\pm.80$ $m^2.g^{-1}$, plus préférentiel-lement de $750\pm70$ à $890\pm80$ $m^2.g^{-1}$. Dans ce mode de réalisation, la teneur en indigo carmine est avantageusement de $10^{-3}$ à 1 $mol.dm^3$, de préférence de $10^{-2}$ à $5\ 10^{-1}$ $mol.dm^3$, plus préférentiellement encore de $10^{-1}$ $mol.dm^3$ à $3\ 10^{-1}$ $mol.dm^3$.

Dans un autre mode de réalisation, la proportion de micropores est de 10% à 80%, de préférence de 20% à 60%, plus préférentiellement de 30% à 40% et la proportion de mésopores est de 20% à 90%, de préférence de 40% à 80%, plus préférentiellement de 60% à 70%. Avantageusement, la matrice de ce mode de réalisation présente une surface spécifique comprise entre $550\pm50$ et $890\pm80$ $m^2.g^{-1}$, notamment entre $650\pm70$ et $890\pm80$ $m^2.g^{-1}$, plus préfé-rentiellement de $750\pm70$ à $890\pm80$ $m^2.g^{-1}$. Dans ce mode de réalisation, la teneur en indigo carmine est avantageu-sement de $10^{-4}$ à $2\ 10^{-3}$ $mol.dm^3$, de préférence de $2\ 10^{-4}$ à $1,5\ 10^{-3}$ $mol.dm^3$, plus préférentiellement encore de $5\ 10^{-4}$ à $10^{-3}$ $mol.dm^3$. Grâce à leurs caractéristiques physico-chimiques particulières décrites plus haut, les matrices sol-gel polyalcoxysilanes nanoporeuses renfermant l'indigo carmine selon l'invention sont capables de piéger sélectivement l'ozone sans présenter les inconvénients de relargage rencontrés avec le charbon actif. De plus, la réaction colorimétrique entre l'ozone et l'indigo carmine permet un suivi visuel de piégeage de l'ozone. Lorsque la matrice devient jaune, ceci indique que la matrice est saturée et l'ozone ne peut plus être retenu dans les micropores de la matrice.

Un objet de l'invention concerne donc également l'utilisation d'une matrice sol-gel polyalcoxysilane nanoporeuse renfermant de l'indigo carmine selon l'invention pour piéger sélectivement l'ozone présent dans l'air. Du fait que la matrice sol-gel protège l'indigo carmine du rayonnement UV, la matrice peut être utilisée aussi bien pour l'air intérieur que pour l'air extérieur.

Dans un aspect de l'invention, la matrice sol-gel polyalcoxysilane nanoporeuse renfermant de l'indigo carmine selon l'invention est utilisée comme filtre d'air pour l'abattement d'ozone. L'invention porte donc également sur un filtre d'abattement d'ozone comprenant une matrice sol-gel polyalcoxysilane nanoporeuse renfermant de l'indigo carmine selon l'invention ainsi que sur un procédé pour filtrer de l'air en piégeant l'ozone présent dans l'air dans les micropores d'une matrice sol-gel polyalcoxysilane nanoporeuse selon l'invention par mise en contact de ladite matrice avec de l'air de façon à séquestrer l'ozone présent dans l'air dans les pores de la dite matrice. Comme décrit ci-dessus, l'ozone réagit avec l'indigo carmine enfermé dans les micropores de la matrice ce qui induit un changement de couleur du bleu foncé via le vert jusqu'au jaune, la couleur jaune indiquant la saturation du filtre.

Pour une utilisation comme filtre d'air pour l'abattement d'ozone, la matrice sol-gel polyalcoxysilane nanoporeuse selon l'invention a avantageusement une teneur en indigo carmine de de $10^{-3}$ à 1 $mol.dm^3$, de préférence de $10^{-2}$ à $5\ 10^{-1}$ $mol.dm^3$, plus préférentiellement encore de $10^{-1}$ $mol.dm^3$ à $3\ 10^{-1}$ $mol.dm^3$.

De plus, la matrice sol-gel polyalcoxysilane nanoporeuse utilisée comme filtre d'air pour l'abattement d'ozone présente une proportion de micropores de 30% à 100%, de préférence de 40% à 90%, plus préférentiellement de 50% à 80% et la proportion de mésopores est de 0% à 70%, de préférence de 10% à 60%, plus préférentiellement de 20% à 50%. Avantageusement, la matrice de ce mode de réalisation présente aussi une surface spécifique comprise entre $550\pm50$ et $890\pm80$ m$^2$.g$^{-1}$, notamment entre $650\pm70$ et $890\pm.80$ m$^2$.g$^{-1}$, plus préférentiellement de $750\pm70$ à $890\pm80$ m$^2$.g$^{-1}$. Dans un mode de réalisation, les matrices selon l'invention sont utilisées en combinaison avec des filtres au charbon actif. Ce mode de réalisation est particulièrement avantageux dans le cas de matrices selon l'invention contenant des groupements phényle et/ou fluorés étant donné que l'hydrophobicité accrue de ces matrices permet d'améliorer le piégeage d'ozone en atmosphère humide. L'utilisation d'une matrice selon l'invention en aval d'un filtre au charbon actif permet de signaler la saturation de ce dernier grâce à la réaction colorimétrique entre l'ozone et l'indigo carmine.

Dans un aspect de l'invention, la matrice sol-gel polyalcoxysilane nanoporeuse renfermant de l'indigo carmine selon l'invention est utilisée dans la détection d'ozone présent dans l'air. L'invention porte donc également sur un capteur d'ozone comprenant une matrice sol-gel polyalcoxysilane nanoporeuse renfermant de l'indigo carmine selon l'invention ainsi que sur un procédé de détection d'ozone présent dans l'air, le procédé comprenant une étape de mise en contact d'une matrice sol-gel polyalcoxysilane nanoporeuse renfermant de l'indigo carmine selon l'invention avec de l'air de façon à séquestrer l'ozone présent dans l'air dans les pores de la dite matrice et une étape de détection de la présence d'ozone grâce à la réaction colorimétrique entre l'ozone et l'indigo carmine. Un changement de couleur de la matrice indique la présence d'ozone. Ce changement de couleur peut être observé à l'œil nu ou à l'aide d'un spectrophotomètre. L'utilisation d'un spectrophotomètre a l'avantage de permettre une quantification de l'ozone présent dans l'air. La détection à l'aide d'un spectrophotomètre est basée sur l'absorbance de l'indigo carmine à 617 nm. Les matrices nanoporeuses selon l'invention étant transparentes, cette mesure peut être réalisée à la fois en mode de transmission ou de réflectance.

Pour une utilisation comme capteur d'ozone, la matrice sol-gel polyalcoxysilane nanoporeuse selon l'invention a avantageusement une teneur en indigo carmine de $10^{-4}$ à $2$ $10^{-3}$. mol.dm$^3$, de préférence de $2$ $10^{-4}$ à $1,5$ $10^{-3}$ mol.dm$^3$, plus préférentiellement encore de $5$ $10^{-4}$ à $10^{-3}$. mol.dm$^3$.

De plus, la matrice sol-gel polyalcoxysilane nanoporeuse utilisé comme capteur d'ozone présente une proportion de micropores de 10% à 80%, de préférence de 20% à 60%, plus préférentiellement de 30% à 40% et la proportion de mésopores est de 20% à 90%, de préférence de 40% à 80%, plus préférentiellement de 60% à 70%. Avantageusement, la matrice de ce mode de réalisation présente une surface spécifique comprise entre $550\pm50$ et $890\pm80$ m$^2$.g$^{-1}$, notamment entre $650\pm70$ et $890\pm80$ m$^2$.g$^{-1}$, plus préférentiellement de $750\pm70$ à $890\pm80$ m$^2$.g$^{-1}$. Naturellement, d'autres modes de réalisation de l'invention auraient pu être envisagés par l'homme du métier sans pour autant sortir du cadre de l'invention définie par les revendications ci-après.

Des exemples non limitatifs de réalisation de l'invention sont décrits ci-après.

## FIGURES

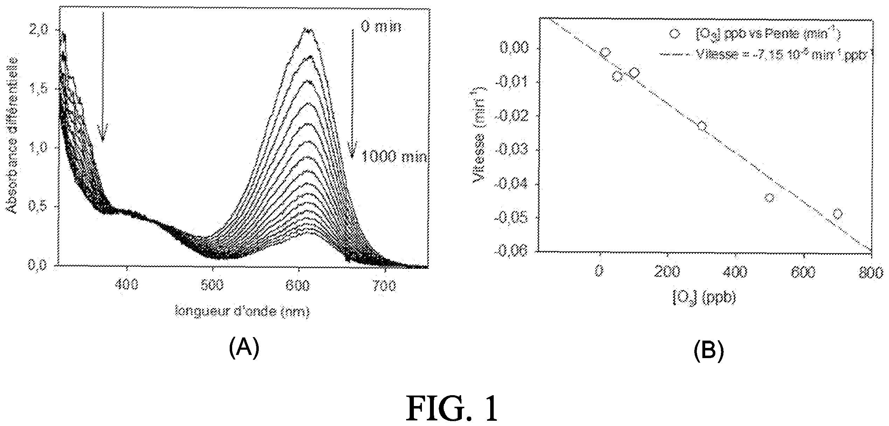

**Figure 1** : Vue en perspective d'une cellule d'exposition permettant la mise en œuvre de mesures par spectrophotométrie sur des rayons transmis par une matrice nanoporeuse lorsque soumise à un rayonnement d'un lampe dans le domaine UV-visible, la cellule présentant un circuit fluidique permettant d'exposer la matrice à un débit d'air maitrisé.

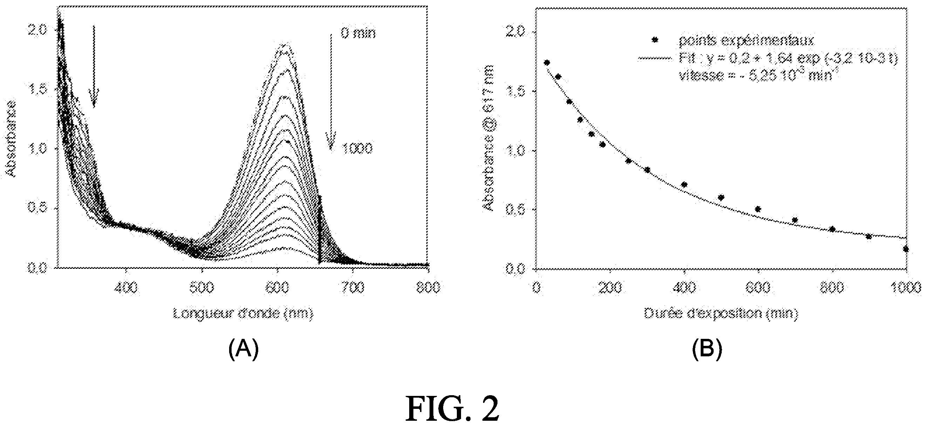

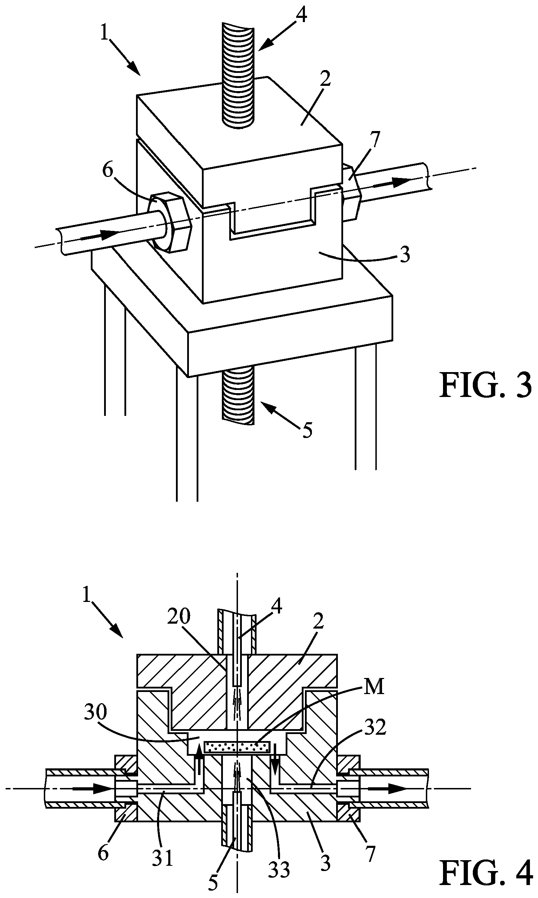

**Figure 2** : Vue de coupe de la cellule d'exposition selon la figure 3, illustrant en détail le circuit de fluide interne, ainsi que le montage optique.

**Figure 3** : A : Evolution spectrale du capteur de l'exemple 1 dopé d'indigo en fonction de la durée d'exposition à un mélange gazeux contenant 50 ppb d'O3 (flux = 200 mL.min$^{-1}$, HR = 5%, T=22°C), B : courbe d'étalonnage du capteur établie à partir des cinétiques monoexponentielles de déclin de l'absorbance à 617 nm en fonction du temps.

**Figure 4** : A : variation spectrale du capteur dopé d'indigo l'exemple 1 en fonction du temps lors de l'exposition à des mélanges gazeux d'ozone (200 ppb) à 50% d'HR et 22°C (flux = 260 mL.min$^{-1}$). B : Cinétique de déclin de l'absorbance du capteur à 617 nm en fonction du temps. La vitesse de disparition de la bande est donnée par la pente à l'origine de la courbe.

## EXEMPLES

## I. Préparation et analyse de matrices sol-gel nanoporeuses

L'analyse de l'isotherme d'adsorption-désorption à l'azote liquide (77 K) avec le modèle DFT (Théorie de la Fonctionnelle de Densité) utilisée pour la détermination de la surface spécifique et du diamètre de pores a été réalisée avec l'Autosorb 1 de Quantachrome.

**Exemple 1 : matrice TMOS dopé d'indigo carmine.**

**Réactifs** : indigo carmine (Sigma Aldrich numéro CAS 860-22-0, Masse molaire= 466.35 g/mol), TMOS (Numéro CAS : 681-84-5, Masse molaire=152.2 g/mol et densité d = 1.023 mg/L), Méthanol ($CH_3OH$ Masse molaire 32,04 g/mol, pureté 98%). Moule en plastique de dimension 12*12 cm et des multipuits de dimension (16*10*4 mm).

**Mode opératoire pour 100 mL de sol :** Dans un ballon, on verse 38,89 mL de TMOS et 42,29 mL de méthanol, et on mélange sous agitation magnétique pendant 1 minute 18,82 mL d'une solution aqueuse d'indigo carmine 0,15 mol.L$^{-1}$ est rajouté au mélange. Le mélange est agité pendant 1heure à température ambiante dans le ballon bien fermé. La solution est versée dans un moule en plastique placé dans un dessiccateur que l'on recouvre de façon hermétique par une membrane d'aluminium. Lorsque la gélification a lieu, la membrane d'aluminium est remplacée par un film poreux. Le séchage est poursuivi dans le même dessiccateur jusqu'à évaporation complète des solvants et la rétractation du monolithe jusqu'à des dimensions finales stables.

Après séchage, on obtient des blocs sol gel ayant la forme de parallélépipèdes bleus foncés de dimensions proches de 6*5*2 mm. La teneur en indigo dans la matrice nanoporeuse est de 0,28 mol.dm$^{-3}$.

La surface spécifique d'adsorption du matériau obtenue avec l'analyse de l'isotherme d'adsorption-désorption à l'azote liquide avec le modèle DFT (Théorie de la Fonctionnelle de Densité) est de $890 \pm 80$ m$^2$.g$^{-1}$. La proportion des micropores est de 84% et le diamètre est centré autour de 1,0 nm. Avec ces tailles de pores, les molécules d'indigo carmine ne peuvent pas sortir de la matrice. L'ozone peut facilement diffuser dans le réseau poreux à l'inverse des molécules de grande taille comme les composés aromatiques monocycliques.

**Exemple 2 : matrice TMOS/PhTMOS dopé d'indigo carmine.**

**Réactifs** : indigo carmine (Sigma Aldrich numéro CAS 860-22-0, Masse molaire= 466.35 g.mol$^{-1}$), TMOS (Numéro CAS : 681-84-5, Masse molaire=152.2 g.mol$^{-1}$ et densité d = 1.023 mg.cm$^{-3}$), PhTMOS (N°CAS : 2996-92-1, Masse molaire = 198,29 g. mol$^{-1}$ et densité = 1,062 g.cm$^{-3}$), Méthanol ($CH_3OH$, Masse molaire 32,04 g.mol$^{-1}$, densité = 0,792 g. cm$^{-3}$, pureté 98%). Moule en plastique multipuits (16*10*4 mm).

**Mode opératoire pour 100 mL de sol :** Dans un ballon, on verse 34,623 mL de TMOS et 4,926 mL de PhTMOS dans 41,837 mL de méthanol, et on mélange sous agitation magnétique pendant 1 minute 18,615 mL d'une solution aqueuse d'indigo carmine 0,15 mol.L$^{-1}$ est rajouté au mélange. Le mélange est agité pendant 1heure à température ambiante dans le ballon bien fermé. La solution est versée dans un moule en plastique placé dans un dessiccateur que l'on recouvre de façon hermétique par une membrane d'aluminium. Lorsque la gélification a lieu, la membrane d'aluminium est remplacée par un film poreux. Le séchage est poursuivi dans le même dessiccateur jusqu'à évaporation complète des solvants et la rétractation du monolithe jusqu'à des dimensions finales stables.

Dans le présent exemple, les proportions molaires des réactifs sont TMOS/PhTMOS/MeOH/H$_2$O : 0,9/0,1/4/4. La teneur en indigo dans la matrice nanoporeuse est de 0,28 mol.dm$^{-3}$.

La surface spécifique d'adsorption du matériau obtenue avec l'analyse de l'isotherme d'adsorption-désorption à l'azote liquide avec le modèle DFT (Théorie de la Fonctionnelle de Densité) est de $800 \pm 50$ m$^2$.g$^{-1}$. La proportion des micropores est de 40% et le diamètre est centré autour de 11 nm. Le diamètre des mésopores est centré autour de 25 Å. Avec ces tailles de pores et la tortuosité du réseau poreux, les molécules d'indigo carmine ne peuvent pas sortir de la matrice. L'ozone peut facilement diffuser dans le réseau poreux à l'inverse des molécules de grande taille comme les composés aromatiques monocycliques.

**Exemple 3 : matrice TMOS/PhTEOS dopé d'indigo carmine.**

**Réactifs** : indigo carmine (Sigma Aldrich numéro CAS 860-22-0, Masse molaire= 466.35 g.mol$^{-1}$), TMOS (Numéro CAS : 681-84-5, Masse molaire=152.2 g.mol$^{-1}$ et densité d = 1.023 mg.cm$^{-3}$), PhTEOS (N°CAS :780-69-8, Masse molaire = 240,37 g.mol$^{-1}$, et densité = 0,996 g.cm$^{-3}$), Méthanol ($CH_3OH$, Masse molaire 32,04 g.mol$^{-1}$, densité = 0,792 g.cm$^{-3}$, pureté 98%). Moule en plastique multipuits (16*10*4 mm).

**Mode opératoire pour 100 mL de Sol :** Dans un ballon, on verse 26,365 mL de TMOS, 14,252 mL de PhTEOS dans 41,097 mL de méthanol, et on mélange sous agitation magnétique pendant 1 minute 18,285 mL d'une solution aqueuse d'indigo carmine 0,15 mol.L$^{-1}$ est rajouté au mélange. Le mélange est agité pendant 1heure à température ambiante dans le ballon bien fermé. La solution est versée dans un moule en plastique placé dans un dessiccateur que l'on recouvre de façon hermétique par une membrane d'aluminium. Lorsque la gélification a lieu, la membrane d'aluminium est remplacée par un film poreux. Le séchage est poursuivi dans le même dessiccateur jusqu'à évaporation complète des solvants et la rétractation du monolithe jusqu'à des dimensions finales stables.

Dans le présent exemple, les proportions molaires des réactifs sont TMOS/PhTEOS/MeOH/H$_2$O :

0,75/0,25/4,3/4,3. La teneur en indigo dans la matrice nanoporeuse est de 0,28 mol.dm$^{-3}$.

La surface spécifique d'adsorption du matériau obtenue avec l'analyse de l'isotherme d'adsorption-désorption à l'azote liquide avec le modèle DFT (Théorie de la Fonctionnelle de Densité) est de 630±50 m$^2$.g$^{-1}$. La proportion des micropores est de 70% et le diamètre est centré autour de 1,1 nm. Le diamètre des mésopores est centré autour de 22 Å. Avec ces tailles de pores et la tortuosité du réseau poreux, les molécules d'indigo carmine ne peuvent pas sortir de la matrice. L'ozone peut facilement diffuser dans le réseau poreux à l'inverse des molécules de grande taille comme les composés aromatiques monocycliques.

**Exemple 4 : matriceTMOS/PhTEOS dopé d'indigo carmine.**

<u>Réactifs</u> : indigo carmine (Sigma Aldrich numéro CAS 860-22-0, Masse molaire= 466.35 g.mol$^{-1}$), TMOS (Numéro CAS : 681-84-5, Masse molaire=152.2 g.mol$^{-1}$ et densité d = 1.023 mg.cm$^{-3}$), PhTEOS (N°CAS :780-69-8, Masse molaire = 240,37 g.mol$^{-1}$, et densité = 0,996 g.cm$^{-3}$), Méthanol (CH$_3$OH, Masse molaire 32,04 g.mol$^{-1}$, densité = 0,792 g.cm$^{-3}$, pureté 98%). Moule en plastique multipuits (16*10*4 mm).

**Mode opératoire pour 100 mL de Sol :** Dans un ballon, on verse 31,90 mL de TMOS, 9,13 mL de PhTEOS dans 40,81 mL de méthanol, et on mélange sous agitation magnétique pendant 1 minute 18,16 mL d'une solution aqueuse d'indigo carmine 0,15 mol.L$^{-1}$ est rajouté au mélange. Le mélange est agité pendant 1heure à température ambiante dans le ballon bien fermé. La solution est versée dans un moule en plastique placé dans un dessiccateur que l'on recouvre de façon hermétique par une membrane d'aluminium. Lorsque la gélification a lieu, la membrane d'aluminium est remplacée par un film poreux. Le séchage est poursuivi dans le même dessiccateur jusqu'à évaporation complète des solvants et la rétractation du monolithe jusqu'à des dimensions finales stables.

Dans le présent exemple, les proportions molaires des réactifs sont TMOS/PhTEOS/MeOH/H$_2$O : 0,85/0,15/4/4. La teneur en indigo dans la matrice nanoporeuse est de 0,28 mol.dm$^{-3}$.

La surface spécifique d'adsorption du matériau obtenue avec l'analyse de l'isotherme d'adsorption-désorption à l'azote liquide avec le modèle DFT (Théorie de la Fonctionnelle de Densité) est de 560±50 m$^2$.g$^{-1}$. La proportion des micropores est de 40% et le diamètre est centré autour de 1,1 nm. Le diamètre des mésopores est centré autour de 24 Å. Avec ces tailles de pores et la tortuosité du réseau poreux, les molécules d'indigo carmine ne peuvent pas sortir de la matrice. L'ozone peut facilement diffuser dans le réseau poreux à l'inverse des molécules de grande taille comme les composés aromatiques monocycliques.

**Exemple 5 : matrice TMOS/3ClTMOS dopé d'indigo carmine.**

<u>Réactifs</u> : indigo carmine (Sigma Aldrich numéro CAS 860-22-0, Masse molaire= 466.35 g.mol$^{-1}$), TMOS (Numéro CAS : 681-84-5, Masse molaire=152.2 g.mol$^{-1}$ et densité d = 1.023 mg.cm$^{-3}$), 3ClTMOS (N°CAS :2530-87-2, pureté 97%, Masse molaire = 198,72 g.mol$^{-1}$, et densité = 1,09 g.cm$^{-3}$), Méthanol (CH$_3$OH, Masse molaire 32,04 g.mol$^{-1}$, densité = 0,792 g.cm$^{-3}$, pureté 98%). Moule en plastique multipuits (16*10*4 mm).

**Mode opératoire pour 100 mL de Sol :** Dans un ballon, on verse 30,488 mL de TMOS, 9,627 mL de 3ClTMOS dans 41,445 mL de méthanol, et on mélange sous agitation magnétique pendant 1 minute 18,44 mL d'une solution aqueuse d'indigo carmine 0,15 mol.L$^{-1}$ est rajouté au mélange. Le mélange est agité pendant 1heure à température ambiante dans le ballon bien fermé. La solution est versée dans un moule en plastique placé dans un dessiccateur que l'on recouvre de façon hermétique par une membrane d'aluminium. Lorsque la gélification a lieu, la membrane d'aluminium est remplacée par un film poreux. Le séchage est poursuivi dans le même dessiccateur jusqu'à évaporation complète des solvants et la rétractation du monolithe jusqu'à des dimensions finales stables.

Dans le présent exemple, les proportions molaires des réactifs sont TMOS/3ClTMOS/MeOH/H$_2$O : 0,8/0,2/4/4. La teneur en indigo dans la matrice nanoporeuse est de 0,28 mol.dm$^{-3}$.

**Exemple 6 : matrice TMOS/3FTMOS dopé d'indigo carmine.**

<u>Réactifs</u> : indigo carmine (Sigma Aldrich numéro CAS 860-22-0, Masse molaire= 466.35 g.mol$^{-1}$), TMOS (Numéro CAS : 681-84-5, pureté 97%, Masse molaire=152.2 g.mol$^{-1}$ et densité d = 1.142 mg.cm$^{-3}$), 3FTMOS (N°CAS :429-60-7, Masse molaire = 218,25 g.mol$^{-1}$, et densité = 0,996 g.cm$^{-3}$), Méthanol (CH$_3$OH, Masse molaire 32,04 g.mol$^{-1}$, densité = 0,792 g.cm$^{-3}$, pureté 98%). Moule en plastique multipuits (16*10*4 mm).

**Mode opératoire pour 100 mL de Sol :** Dans un ballon, on verse 25,854 mL de TMOS, 16,108 mL de 3FTMOS dans 40,167 mL de méthanol, et on mélange sous agitation magnétique pendant 1 minute 17,872 mL d'une solution aqueuse d'indigo carmine 0,15 mol.L$^{-1}$ est rajouté au mélange. Le mélange est agité pendant 1heure à température ambiante dans le ballon bien fermé. La solution est versée dans un moule en plastique placé dans un dessiccateur que l'on recouvre de façon hermétique par une membrane d'aluminium. Lorsque la gélification a lieu, la membrane d'aluminium est remplacée par un film poreux. Le séchage est poursuivi dans le même dessiccateur jusqu'à évaporation complète des solvants et la rétractation du monolithe jusqu'à des dimensions finales stables.

Dans le présent exemple, les proportions molaires des réactifs sont TMOS/3FTMOS/MeOH/H$_2$O : 0,7/0,3/4/4. La teneur en indigo dans la matrice nanoporeuse est de 0,28 mol.dm$^{-3}$.

La surface spécifique d'adsorption du matériau obtenue avec l'analyse de l'isotherme d'adsorption-désorption à l'azote liquide avec le modèle DFT (Théorie de la Fonctionnelle de Densité) est de 460 $\pm$ 60 m$^2$.g$^{-1}$. La proportion des micropores est de 40 % et le diamètre est centré autour de 1,6 nm. Avec ces tailles de pores, les molécules d'indigo carmine ne peuvent pas sortir de la matrice. L'ozone peut facilement diffuser dans le réseau poreux à l'inverse des molécules de grande taille comme les composés aromatiques monocycliques.

## Exemple 7 : matrice TMOS/APTES dopé d'indigo carmine.

**Réactifs** : indigo carmine (Sigma Aldrich numéro CAS 860-22-0, Masse molaire= 466.35 g.mol$^{-1}$), TMOS (Numéro CAS : 681-84-5, pureté 97%, Masse molaire=152.2 g.mol$^{-1}$ et densité d = 1.142 mg.cm$^{-3}$), APTES (N°CAS : 919-30-2, Masse molaire = 208,33g.mol$^{-1}$, et densité = 0,933 g.cm$^{-3}$ ), Méthanol (CH$_3$OH, Masse molaire 32,04 g.mol$^{-1}$, densité = 0,792 g.cm$^{-3}$, pureté 98%). Moule en plastique multipuits (16*10*4 mm).

**Mode opératoire pour 100 mL de Sol :** Dans un ballon contenant 45,582 mL de méthanol maintenu à basse température (-25°C), on verse 33,91 mL de TMOS et 1,574 mL de APTES et on mélange sous agitation magnétique pendant 2 minutes 16,934 mL d'une solution aqueuse d'indigo carmine 0,15 mol.L$^{-1}$ est rajouté au mélange. Le mélange est agité pendant 2 minutes et la solution est versée dans un moule en plastique, puis placé dans un dessiccateur que l'on recouvre de façon hermétique par une membrane d'aluminium. Lorsque la gélification a lieu, la membrane d'aluminium est remplacée par un film poreux. Le séchage est poursuivi dans le même dessiccateur jusqu'à évaporation complète des solvants et la rétractation du monolithe jusqu'à des dimensions finales stables.

Dans le présent exemple, les proportions molaires des réactifs sont TMOS/APTES/MeOH/H$_2$O : 0,97/0,03/5/4. La teneur en indigo dans la matrice nanoporeuse est de 0,28 mol.dm$^{-3}$.

La surface spécifique d'adsorption du matériau obtenue avec l'analyse de l'isotherme d'adsorption-désorption à l'azote liquide avec le modèle DFT (Théorie de la Fonctionnelle de Densité) est de 820$\pm$70 m$^2$.g$^{-1}$. La proportion des micropores est de 20% et le diamètre est centré autour de 17 Å. La proportion des mésopores est de 80% et le diamètre est centré autour de 27 Å.

## II. Tests de piégeage de l'ozone.

Les tests de piégeage des matrices dopés d'indigo exposés à O$_3$ ont été réalisés pour quantifier l'efficacité de piégeage et ainsi démontrer leur utilité comme filtre d'abattement d'ozone. Les matrices sous forme de parallélépipèdes obtenues aux Exemples 1 à 6 ont été grossièrement broyées et tamisées (passage par 2 tamis) pour l'obtention de granulés de taille millimétrique.

Les filtres ont été préparés sous forme de seringues en plastique (6 mL de volume) munis de deux embouts dont l'un est connecté via un tuyau au générateur de mélange gazeux. L'embout de sortie de la seringue est connecté à un tuyau qui va dans une hotte d'aspiration. Ces seringues ont été remplies des différents granulés et exposés à un mélange d'air contenant de l'ozone.

Le banc d'analyse comporte des débitmètres massiques et un système d'injection d'humidité pour permettre l'exposition des filtres en fonction du débit du mélange gazeux à diverses humidités. L'ozone est généré avec un générateur d'O$_3$ et sa concentration est mesurée en amont et en aval du filtre grâce à un analyseur d'O$_3$.

Le débit du flux en amont de la cartouche est de 2L/min et celui en aval a été mesuré pour chaque cartouche afin de vérifier que la perte de charge est à peu près la même pour toutes les cartouches. Le taux de piégeage, $\tau$, est mesuré pour chaque type de filtre en fonction des paramètres d'exposition. Il est défini selon :

$$\tau = \frac{[O3_{amont}] - [O3_{aval}]}{[O3_{amont}]} 100\%$$

La concentration d'ozone ([O3$_{amont}$]) choisie, ~40 ppb, correspond à une valeur moyenne de la teneur d'ozone dans l'air extérieur. L'effet de l'humidité du flux de mélange gazeux (HR% = 50 et 72) a également été étudié. Les performances de piégeage ont été comparées pour des durées d'exposition allant de la minute à 480 heures et jusqu'à 20 jours pour quelques essais. Par ailleurs, une comparaison des performances de piégeage a été réalisée avec un piège à ozone commercial à base de KI et un piège à base de charbon actif.

Les données obtenues sont regroupées dans les tableaux suivants.

**Tableau 1. Performances comparées de piégeage d'O$_3$ à faible concentration pour divers matériaux**

| Flux amont = 2 L/min, [O$_3$]$_{amont}$ = 42±3 ppb, HR = 50 % | | | |
|---|---|---|---|
| Durée (h) | Taux de piégeage | | |
| | Mat 1 (1,79 g) | Mat 2 (1,73 g) | KI commercial (1,5g) |
| 1 | 100 % | 100 % | 80 % |
| 24 | 94% | 100 % | 61 % |
| 48 | 82 % | 100 % | 48 % |
| 72 | 72 % | 100 % | |
| 168 | 59 % | 94 % | |
| 384 | 30 % | 64 % | |
| 480 | 22 % | 55 % | |
| Débit aval (L/min) | 1,32 | 1,22 | 1,44 |

Les matériaux Mat 1 (Exemple 1) et Mat 2 (Exemple 2) ont tous deux des petits pores, mais Mat 2 est plus hydrophobe, ce qui explique sa meilleure performance et durée de vie. La comparaison avec le produit commercial contenant du KI montre la supériorité des matériaux sol-gel qui allie à la fois une grande surface spécifique d'adsorption propice au piégeage d'O$_3$.

Des tests à forte humidité relative, HR = 72% ont été également réalisés pour comparer l'efficacité de piégeage d'O$_3$ dans des conditions où la concentration de vapeur d'eau est très élevée et pourrait contribuer à boucher les pores du filtre et diminuer son efficacité.

**Tableau 2. Performances comparées de piégeage d'O3 à forte humidité d'un filtre nanoporeux dopé d'indigo avec un filtre de charbon actif**

| Flux amont = 2 L/min, HR = 72 % | | | |
|---|---|---|---|
| Durée expo (h) | [O3] (ppb) | Taux de piégeage | |
| | | Mat 2 (1,79 g) | CA RBAA3 (1,75 g) |
| 24 | 43±5 | 100 % | 100 % |
| 48 | 43±5 | 100 % | 90 % |
| 72 | 43±5 | 98 % | 89 % |
| 96 | 43±5 | 92 % | 79 % |
| Débit aval (L/min) | | 1,27 | 1,16 |

Le charbon actif choisi (Norit RBAA-3 en bâtonnet, Fluka) présente de très petits pores (diamètre de pore < 11Å) et une surface spécifique d'adsorption de 960 m$^2$.g$^{-1}$. Les données collectées dans le tableau 2 montrent que MAT 2 piège mieux l'ozone que le charbon actif à forte humidité.

Des tests ont été également réalisés à très forte concentration d'ozone

**Tableau 3 Performances comparées de piégeage d'O3 à forte concentration d'O3 pour différents matériaux dopés d'indigo**

| Flux amont = 2 L/min, [O$_3$]$_{amont}$ = 475±10 ppb, HR = 50 % | | | | | |
|---|---|---|---|---|---|
| Durée (min) | Taux de piégeage | | | | |
| | Mat 1 (1,79 g) | Mat 2 (1,79 g) | Mat 3 (1,79g) | Mat 6 (1,79g) | Mat 7 (1,79g) |
| 10 | 99,5 % | 100 % | 100 % | 90,8 % | 100 % |
| 20 | 99,5 % | 97,3 % | 99,4 % | 89,2 % | 97,3% |
| 180 | 74,0 % | - | 57,6 % | 55,0 % | 50,0 % |

(suite)

| Flux amont = 2 L/min, $[O_3]_{amont}$ = 475±10 ppb, HR = 50 % | | | | | |
|---|---|---|---|---|---|
| Durée (min) | Taux de piégeage | | | | |
| | Mat 1 (1,79 g) | Mat 2 (1,79 g) | Mat 3 (1,79g) | Mat 6 (1,79g) | Mat 7 (1,79g) |
| 300 | 51,0 % | 71,2 % | 52,0 % | 39,0 % | 20,0 % |
| 1440 | 22,0 % | 26,0 % | 31,0 % | - | 2,0 % |
| Débit aval (L/min) | 1,32 | 1,22 | 1,22 | 1,30 | 1,42 |

Les matériaux Mat 1, Mat 2 et Mat 3 qui ont une grande proportion de micropores ont les meilleures efficacités de piégeage d'$O_3$ à forte concentration du polluant. Le Mat 7 est celui qui présente un pourcentage élevé de mésopores et est le moins performant.

Par ailleurs, un des avantages du filtre à base d'indigo carmine est le changement de couleur visible à l'œil nu qui permet d'apprécier son état de saturation. En effet, la couleur du filtre change au fur et mesure du piégeage de l'ozone du bleu via le vert jusqu'au jaune. Pour le matériau 2, le filtre n'était toujours pas saturé après 20 jours d'exposition à un flux de 2L/min avec une teneur d'$O_3$ de 42±3 ppb, à une humidité relative de 50%.

### III. Mesure de l'ozone avec la matrice de l'Exemple 1

La matrice (capteur) est positionnée dans une cellule d'exposition comportant un circuit fluidique au travers duquel va passer le mélange de flux gazeux contenant de l'ozone. La cellule d'exposition est munie de deux fenêtres optiques par lesquelles passe la lumière d'analyse véhiculée par des fibres optiques et provenant d'une lampe UV-visible. Après traversée du capteur, la lumière transmise est collectée avec un spectrophotomètre miniature Ocean-Optics et les données collectées sont transmises à l'ordinateur.

Cette cellule d'exposition 1 est illustrée aux figures 1 et 2 et comprend un corps 1 qui se compose de deux parties 2,3, respectivement supérieure et inférieure. La matrice nano poreuse M est positionnée par un opérateur dans une cavité 30 formée sur la partie inférieure 3, lorsque les deux parties 2,3 sont démontées. Cette cavité 30 est ensuite fermée de manière étanche au gaz par l'assemblage de la partie supérieure 2 sur la partie inférieure 3.

Le montage optique comprend une première fenêtre optique, matérialisée par un usinage traversant 20 de la partie supérieure 2, débouchant dans la cavité 30, et au travers de laquelle passe la lumière véhiculée par les fibres optiques 4 et provenant de la lampe UV-visible. Une deuxième fenêtre optique, matérialisée par un usinage traversant 33 de la partie inférieure 3 permet la collecte des rayons transmis par la matrice nano poreuse. La collecte s'effectue par des fibres optiques 5 qui acheminent les rayonnements collectés jusqu'au spectrophotomètre miniature Ocean-Optics.

Le circuit fluidique est matérialisé sur la partie inférieure 3 et comprend un premier raccord 6 connecté à un tuyau d'alimentation d'air, qui débouche dans un usinage 31 traversant de la partie inférieure 3 acheminant l'air provenant du tuyau jusqu'à la cavité 30. Un autre usinage 32 traversant de la partie inférieure 3 permet l'évacuation du gaz, depuis la cavité 30 jusqu'à un second raccord 7, connecté à un tuyau d'évacuation.

Un spectre est collecté avant exposition. Durant l'exposition, les spectres sont collectés tous les 10 min pendant 48 H.

Le flux gazeux contenant de l'ozone à différentes concentrations provient d'un générateur d'ozone MEGATEC modèle 165. La concentration a été variée de 3 à 1000 ppb. La concentration d'ozone a été préalablement contrôlée avec un analyseur d'ozone modèle 49C de MEGATEC. Le débit d'exposition a été fixé à 260 mL.min$^{-1}$, est contrôlée avec un débitmètre massique. L'humidité du mélange est apportée avec un second flux d'azote humidifié à 100% (par barbotage dans un flacon d'eau) auquel est mélangé le flux d'ozone.

Un exemple de spectres collectés pendant l'exposition de la matrice de l'exemple 1 à un mélange gazeux contenant 50 ppb d'ozone (flux = 200 mL.min$^{-1}$, HR = 5%, T=22°C) est montré à la figure 3.

Les cinétiques de déclin ont été établies à partir des courbes de variation d'absorbance à 617 nm en fonction du temps pour diverses concentrations d'ozone. Les vitesses de réaction ont été déduites des cinétiques monoexponentielles de déclin et reportées en fonction de la concentration d'ozone. La courbe d'étalonnage ainsi établie montre une variation linéaire de la vitesse de perte de coloration avec la concentration d'ozone avec un coefficient de -7,15 10$^{-5}$ min$^{-1}$.ppb$^{-1}$. La limite de détection est de 3 ppb pour une exposition de 60 min à un flux de 260 mL.min$^{-1}$. La sensibilité d'un tel capteur pourrait encore être améliorée en augmentant le flux d'exposition du capteur.

La variation de couleur du capteur d'ozone est également visible à l'œil nu. Le capteur initialement bleu (indigo carmine seul) devient vert en présence d'isatine de couleur jaune résultant de la réaction de l'indigo carmine avec l'ozone (bleu+ jaune =vert), puis jaune en fin d'exposition lorsque tout l'indigo carmine a disparu au profit de l'isatine.

**IV. Mesure de l'ozone avec la matrice de l'Exemple 1 en présence d'humidité à hauteur de 50%**

Il a été procédé comme au point III mais le taux d'humidité relative du flux d'exposition était de 50%. Les résultats obtenus sont reportés sur la figure 4. On observe que la réponse du capteur est bien sensible et reproductible même avec ce taux élevé d'humidité de l'ordre de 50%.

La courbe de calibration établie pour une humidité relative de 50% montre que la réponse du capteur est plus lente avec une vitesse de -2,6 $10^{-5}$ ppb$^{-1}$.min$^{-1}$, soit environ 2,8 fois plus lente que celle obtenue à 5% d'HR. L'eau interfère en diminuant le piégeage de l'ozone dans le capteur nanoporeux.

**REFERENCES**

[1] Clifford Weisel, Charles J. Weschler, Kris Mohan, Jose Vallarino John D. Spengler, Ozone and Ozone Byproducts in the Cabins of Commercial Aircraft, Environ. Sci. Technol., dx.doi.org/10.1021/es3046795

[2] P. Huré, X. Rousselin, l'émission d'ozone par les photocopieurs et imprimantes laser, INRS, Ed. N°1422, 1997.

[3] A. Ginestet, D. Pugnet, L'efficacité des filtres moléculaires pour la ventilation des bâtiments, Centre technique des industries aérauliques et thermique, Juin/Juillet 07, bimestriel.

[4] Todor Batakliev, Vladimir Georgiev, Metody Anachkov, Slavcho Rakovsky, Gennadi E. Zaikov, Ozone decomposition, Interdisciplinary Toxicology. 2014; Vol. 7(2): 47-59

[5] J.P Viricelle, A. Pauly, L Mazet, J.Brunet, M Bouvet, C. Varenne, C.Pijolat, Selectivity improvement of semi-conducting gas sensors by selective filter for atmospheric polluants detection, Materials Science and Enginnering C 26(2006) 186-195.

[6] M. Alexy, G. Voss, J. Heinze, Optochemical sensor for determining ozone based on novel soluble indigo dyes immobilised in a highly permeable polymeric film. Analytical and Bioanalytical Chemistry, 382 (2005), 1628-1641.

[7] M. Yasuko Yamada, Measurement of ambient ozone using newly developed porous glass sensor, Sensors and Actuators B 126 (2007) 485-491.

[8.] G. Kiriakidis, J. Kortidis, K. Moschovis, D. Dovinos, On the Road to Inexpensive, sub-ppb, Room temperature Ozone Sensing, Sensor Lett. Vol.6, No6, (2008), 812-816.

[9.] J. L. Lambert, Y. C. Chiang, J. V. Paukstelis, Colorimetric detector for ozone and method of preparation, US 4,859,607 du 22 Août 1989.

[10.] Anna C. Franklin, et al., "Ozone Measurement in South Carolina Using Passive Sampler", Journal of the Air & Waste Measurement Association, Vol. 54, pp. 1312-1320, 2004, doi: 10.1080/10473289.2004.10470997.

[11.] M. Alexy, G. Voss, J. Heinze, Optochemical sensor for determining ozone based on novel soluble indigo dyes immobilised in a highly permeable polymeric film. Analytical and Bioanalytical Chemistry, 382 (2005), 1628-1641.

[12.] M. Yasuko Yamada, Measurement of ambient ozone using newly developed porous glass sensor, Sensors and Actuators B 126 (2007) 485-491.

[13.] General Information on Vycor Glass No. 7930 (Corning Glass Works, Corning, NY14830).

[14.] Takashi Miwa, Yasuko Maruo, Jiro Nakamura, Tatsuya Kunioka, Seizo Sakata, Ozone detecting element, WO 2008056513 A1, 15 Mai 2008.

[15.] Gago-Ferrero P, Demeestere K, Silvia Díaz-Cruz M, Barceló D., Ozonation and peroxone oxidation of benzophenone-3 in water: effect of operational parameters and identification of intermediate products, .Sci Total Environ. 2013 Jan 15; 443 : 209-17. doi:10.1016/j.scitotenv.2012.10.006.

[16.] Fernando J. Beltran, Ozone reaction kinetics in water and waste water systems, Taylor & Francis e-library, 2005.

**Revendications**

**1.** Procédé de préparation d'une matrice sol-gel polyalcoxysilane nanoporeuse renfermant de l'indigo carmine, ledit procédé comprenant les étapes suivantes :

a) synthèse d'un gel à partir de tétraméthoxysilane ou d'un mélange de tétraméthoxysilane et d'un autre précurseur organosilicé choisi parmi le phényltriméthoxysilane, le phényltriéthoxysilane, un fluoroalkyltriméthoxysilane, un fluoroalkyltriéthoxysilane, un chloroalkylméthoxysilane, un chloroalkyléthoxysilane, un aminopropyltriéthoxysilane et leurs mélanges, la synthèse étant effectuée en milieu aqueux en présence d'un solvant organique polaire et de l'indigo carmine à une température allant de 20 à 70. °C

b) séchage du gel obtenu à l'étape a) de façon à obtenir une matrice sol-gel polyalcoxysilane nanoporeuse renfermant de l'indigo carmine.

2. Procédé selon la revendication précédente, **caractérisé en ce que** la synthèse du gel à l'étape a) est une synthèse monotope.

3. Procédé selon l'une quelconque des revendications précédentes, **caractérisé en ce que** la synthèse du gel à l'étape a) est réalisée à partir de tétraméthoxysilane ou d'un mélange de de tétraméthoxysilane et d'un autre précurseur organosilicé choisi parmi le phényltriméthoxysilane (PhTMOS), le phényltriéthoxysilane (PhTEOS), un fluoropropyltriméthoxysilane, un fluoropropyltriéthoxysilane, un chloropropylméthoxysilane, un chloropropyléthoxysilane, un aminopropyltriéthoxysilane et leurs mélanges.

4. Procédé selon la revendication 3, **caractérisé en ce que** la synthèse du gel à l'étape a) est réalisée à partir de tétraméthoxysilane.

5. Procédé selon la revendication 3, **caractérisé en ce que** la synthèse du gel à l'étape a) est réalisée à partir d'un mélange de tétraméthoxysilane et d'un autre précurseur organosilicé choisi parmi le phényltriméthoxysilane, le phényltriéthoxysilane, un fluoropropyltriméthoxysilane, un chloropropylméthoxysilane, un aminopropyltriéthoxysilane et leurs mélanges.

6. Procédé selon la revendication 5, **caractérisé en ce que** la synthèse du gel à l'étape a) est réalisée à partir d'un mélange de tétraméthoxysilane et d'un autre précurseur organosilicé choisi parmi le phényltriméthoxysilane, le phényltriéthoxysilane et le triméthoxy(3,3,3-trifluoropropyl)silane, le (3-chloropropyl)triméthoxysilane et le (3-aminopropyl)triéthoxysilane.

7. Procédé selon la revendication 5, **caractérisé en ce que** la synthèse du gel à l'étape a) est réalisée à partir d'un mélange de tétraméthoxysilane et du phényltriméthoxysilane ou du phényltriéthoxysilane.

8. Procédé selon la revendication 3, **caractérisé en ce que** la synthèse du gel à l'étape a) est réalisée à partir d'un mélange de tétraméthoxysilane et d'un autre précurseur organosilicé choisi parmi le phényltriméthoxysilane, le phényltriéthoxysilane, un fluoroalkyltriméthoxysilane, un chloroalkyltriméthoxysilane et leurs mélanges.

9. Procédé selon la revendication 3, 5, 6, 7 ou 8, **caractérisé en ce que** les proportions molaires de tétraméthoxysilane/autre précurseur organosilicé sont comprises entre 0,97/0,03 et 0,6/0,4, de préférence entre 0,9/0,1 et 0,7/0,3.

10. Procédé selon l'une quelconque des revendications précédentes, **caractérisé en ce que** le solvant organique polaire est du méthanol.

11. Procédé selon l'une quelconque des revendications précédentes, **caractérisé en ce que** les proportions molaires de solvant organique polaire et d'eau sont comprises respectivement entre 4 et 10, de préférence entre 4 et 7 et plus préférentiellement entre 4 et 5.

12. Matrice sol-gel polyalcoxysilane nanoporeuse renfermant de l'indigo carmine obtenue à partir de tétraméthoxysilane ou d'un mélange de tétraméthoxysilane et d'un autre précurseur organosilicé choisi parmi le phényltriméthoxysilane, le phényltriéthoxysilane, un fluoroalkyltriméthoxysilane, un fluoroalkyltriéthoxysilane, un chloroalkylméthoxysilane, un chloroalkyléthoxysilane, un aminopropyltriéthoxysilane et leurs mélanges, **caractérisée en ce qu'**elle présente une surface spécifique de $550 \pm 60$ m$^2$.g$^{-1}$ à $890 \pm 80$ m$^2$.g$^{-1}$, de préférence de $650 \pm 60$ m$^2$.g$^{-1}$ à $890 \pm 80$ m$^2$.g$^{-1}$ et plus préférentiellement encore de $750 \pm 70$ m$^2$.g$^{-1}$ à $890 \pm 80$m$^2$.g$^{-1}$, et une proportion de micropores supérieure à 30 %, de préférence supérieure à 40 % et plus préférentiellement encore supérieure à 60 %, la surface spécifique et la distribution des tailles de pores étant déterminées par analyse de l'isotherme d'adsorption à l'azote liquide avec le modèle DFT, le terme « DFT » signifiant Théorie de la Fonctionnelle de Densité.

13. Utilisation d'une matrice sol-gel polyalcoxysilane nanoporeuse renfermant de l'indigo carmine selon la revendication 12 pour piéger sélectivement l'ozone présent dans l'air.

14. Utilisation selon la revendication 13 **caractérisée en ce que** la matrice est utilisée comme filtre d'air pour l'abattement d'ozone.

**15.** Utilisation selon la revendication 13 **caractérisée en ce que** la matrice est utilisée dans la détection d'ozone présent dans l'air.

**16.** Filtre d'abattement d'ozone, **caractérisé en ce qu'**il comprend une matrice sol-gel polyalcoxysilane nanoporeuse renfermant de l'indigo carmine selon la revendication 12.

**17.** Capteur d'ozone, **caractérisé en ce qu'**il comprend une matrice sol-gel polyalcoxysilane nanoporeuse renfermant de l'indigo carmine selon la revendication 12.

**Patentansprüche**

**1.** Verfahren zur Herstellung einer nanoporösen Polyalkoxysilan-Sol-Gel-Matrix, die Indigokarmin einschließt, wobei das Verfahren die folgenden Schritte umfasst:

a) Synthese eines Gels ausgehend von Tetramethoxysilan oder einer Mischung aus Tetramethoxysilan und einem anderen Organosilizium-Vorläufer, ausgewählt aus Phenyltrimethoxysilan, Phenyltriethoxysilan, einem Fluoralkyltrimethoxysilan, einem Fluoralkyltriethoxysilan, einem Chloralkylmethoxysilan, einem Chloralkylethoxysilan, einem Aminopropyltriethoxysilan und Mischungen davon, wobei die Synthese in einer wässrigen Umgebung in Gegenwart eines polaren organischen Lösungsmittels und Indigokarmin bei einer Temperatur im Bereich von 20 bis 70 °C durchgeführt wird
b) Trocknung des in Schritt a) erhaltenen Gels auf solche Weise, dass eine nanoporöse Polyalkoxysilan-Sol-Gel-Matrix, die Indigokarmin einschließt, erhalten wird.

**2.** Verfahren nach dem vorhergehenden Anspruch, **dadurch gekennzeichnet, dass** die Synthese des Gels in Schritt a) eine Monotopsynthese ist.

**3.** Verfahren nach einem der vorhergehenden Ansprüche, **dadurch gekennzeichnet, dass** die Synthese des Gels in Schritt a) ausgehend von Tetramethoxysilan oder einer Mischung aus Tetramethoxysilan und einem anderen Organosilizium-Vorläufer, ausgewählt aus Phenyltrimethoxysilan (PhTMOS), Phenyltriethoxysilan (PhTEOS), einem Fluorpropyltrimethoxysilan, einem Fluorpropyltriethoxysilan, einem Chlorpropylmethoxysilan, einem Chlorpropylethoxysilan, einem Aminopropyltriethoxysilan und Mischungen davon, durchgeführt wird.

**4.** Verfahren nach Anspruch 3, **dadurch gekennzeichnet, dass** die Synthese des Gels in Schritt a) ausgehend von Tetramethoxysilan durchgeführt wird.

**5.** Verfahren nach Anspruch 3, **dadurch gekennzeichnet, dass** die Synthese des Gels in Schritt a) ausgehend von einer Mischung aus Tetramethoxysilan und einem anderen Organosilizium-Vorläufer, ausgewählt aus Phenyltrimethoxysilan, Phenyltriethoxysilan, einem Fluoropropyltrimethoxysilan, einem Chlorpropylmethoxysilan, einem Aminopropyltriethoxysilan und Mischungen davon, durchgeführt wird.

**6.** Verfahren nach Anspruch 5, **dadurch gekennzeichnet, dass** die Synthese des Gels in Schritt a) ausgehend von einer Mischung aus Tetramethoxysilan und einem anderen Organosilizium-Vorläufer, ausgewählt aus Phenyltrimethoxysilan, Phenyltriethoxysilan und Trimethoxy(3,3,3-trifluorpropyl)silan, (3-Chlorpropyl)trimethoxysilan und (3-Aminopropyl)triethoxysilan, durchgeführt wird.

**7.** Verfahren nach Anspruch 5, **dadurch gekennzeichnet, dass** die Synthese des Gels in Schritt a) ausgehend von einer Mischung aus Tetramethoxysilan und Phenyltrimethoxysilan oder Phenyltriethoxysilan durchgeführt wird.

**8.** Verfahren nach Anspruch 3, **dadurch gekennzeichnet, dass** die Synthese des Gels in Schritt a) ausgehend von einer Mischung aus Tetramethoxysilan und einem anderen Organosilizium-Vorläufer, ausgewählt aus Phenyltrimethoxysilan, Phenyltriethoxysilan, einem Fluoralkyltrimethoxysilan, einem Chloralkyltrimethoxysilan und Mischungen davon, durchgeführt wird.

**9.** Verfahren nach Anspruch 3, 5, 6, 7 oder 8, **dadurch gekennzeichnet, dass** die molaren Verhältnisse von Tetramethoxysilan/anderer Organosilizium-Vorläufer zwischen 0,97/0,03 und 0,6/0,4, vorzugsweise zwischen 0,9/0,1 und 0,7/0,3, liegen.

**10.** Verfahren nach einem der vorhergehenden Ansprüche, **dadurch gekennzeichnet, dass** das polare organische Lösungsmittel Methanol ist.

**11.** Verfahren nach einem der vorhergehenden Ansprüche, **dadurch gekennzeichnet, dass** die molaren Verhältnisse des polaren organischen Lösungsmittels und des Wassers jeweils zwischen 4 und 10, vorzugsweise zwischen 4 und 7 und noch bevorzugter zwischen 4 und 5 liegen.

**12.** Nanoporöse Polyalkoxysilan-Sol-Gel-Matrix, die Indigokarmin einschließt, die ausgehend von Tetramethoxysilan oder einer Mischung aus Tetramethoxysilan und einem anderen Organosilizium-Vorläufer, ausgewählt aus Phenyltrimethoxysilan, Phenyltriethoxysilan, einem Fluoralkyltrimethoxysilan, einem Fluoralkyltriethoxysilan, einem Chloralkylmethoxysilan, einem Chloralkylethoxysilan, einem Aminopropyltriethoxysilan und Mischungen davon, erhalten ist, **dadurch gekennzeichnet, dass** sie eine spezifische Oberfläche von $550 \pm 60$ m$^2$.g$^{-1}$ bis $890 \pm 80$ m$^2$.g$^{-1}$, vorzugsweise von $650 \pm 60$ m$^2$.g$^{-1}$ bis $890 \pm 80$ m$^2$.g$^{-1}$ und noch bevorzugter von $750 \pm 70$ m$^2$.g$^{-1}$ bis $890 \pm 80$ m$^2$.g$^{-1}$ und ein Verhältnis an Mikroporen von mehr als 30 %, vorzugsweise mehr als 40 % und noch bevorzugter mehr als 60 % aufweist, wobei die spezifische Oberfläche und die Porengrößenverteilung durch Analyse der Adsorptionsisotherme von flüssigem Stickstoff mit dem DFT-Modell bestimmt worden sind, wobei der Begriff "DFT" Dichtefunktionstheorie bedeutet.

**13.** Verwendung einer nanoporösen Polyalkoxysilan-Sol-Gel-Matrix, die Indigokarmin einschließt, nach Anspruch 12 zum selektiven Binden von in der Luft vorhandenem Ozon.

**14.** Verwendung nach Anspruch 13, **dadurch gekennzeichnet, dass** die Matrix als Luftfilter für die Entfernung von Ozon verwendet wird.

**15.** Verwendung nach Anspruch 13, **dadurch gekennzeichnet, dass** die Matrix bei dem Nachweis von in der Luft vorhandenem Ozon verwendet wird.

**16.** Filter zum Binden von Ozon, **dadurch gekennzeichnet, dass** er eine nanoporöse Polyalkoxysilan-Sol-Gel-Matrix, die Indigokarmin einschließt, nach Anspruch 12 umfasst.

**17.** Ozonsensor, **dadurch gekennzeichnet, dass** er eine nanoporöse Polyalkoxysilan-Sol-Gel-Matrix, die Indigokarmin einschließt, nach Anspruch 12 umfasst.

**Claims**

**1.** A process for preparing a nanoporous polyalkoxysilane sol-gel matrix containing carmine indigo, said process comprising the following steps:

a) synthesis of a gel from tetramethoxysilane or from a mixture of tetramethoxysilane and of another organosilicon precursor chosen from phenyltrimethoxysilane, phenyltriethoxysilane, a fluoroalkyltrimethoxysilane, a fluoroalkyltriethoxysilane, a chloroalkylmethoxysilane, a chloroalkylethoxysilane, an aminopropyltriethoxysilane, and mixtures thereof, the synthesis being performed in aqueous medium in the presence of a polar organic solvent and of carmine indigo at a temperature ranging from 20 to 70°C,

b) drying of the gel obtained in step a) so as to obtain a nanoporous polyalkoxysilane sol-gel matrix containing carmine indigo.

**2.** The process as claimed in the preceding claim, **characterized in that** the synthesis of the gel in step a) is a one-pot synthesis.

**3.** The process as claimed in any one of the preceding claims, **characterized in that** the synthesis of the gel in step a) is performed using tetramethoxysilane or a mixture of tetramethoxysilane and of another organosilicon precursor chosen from phenyltrimethoxysilane (PhTMOS), phenyltriethoxysilane (PhTEOS), a fluoropropyltrimethoxysilane, a fluoropropyltriethoxysilane, a chloropropylmethoxysilane, a chloropropylethoxysilane, an aminopropyltriethoxysilane, and mixtures thereof.

**4.** The process as claimed in claim 3, **characterized in that** the synthesis of the gel in step a) is performed using tetramethoxysilane.

5. The process as claimed in claim 3, **characterized in that** the synthesis of the gel in step a) is performed using a mixture of tetramethoxysilane and of another organosilicon precursor chosen from phenyltrimethoxysilane, phenyltriethoxysilane, a fluoropropyltrimethoxysilane, a chloropropylmethoxysilane, an aminopropyltriethoxysilane, and mixtures thereof.

6. The process as claimed in claim 5, **characterized in that** the synthesis of the gel in step a) is performed using a mixture of tetramethoxysilane and of another organosilicon precursor chosen from phenyltrimethoxysilane, phenyltriethoxysilane, trimethoxy(3,3,3-trifluoropropyl)silane, (3-chloropropyl)trimethoxysilane and (3-aminopropyl)triethoxysilane.

7. The process as claimed in claim 5, **characterized in that** the synthesis of the gel in step a) is performed using a mixture of tetramethoxysilane and of phenyltrimethoxysilane or of phenyltriethoxysilane.

8. The process as claimed in claim 3, **characterized in that** the synthesis of the gel in step a) is performed using a mixture of tetramethoxysilane and of another organosilicon precursor chosen from phenyltrimethoxysilane, phenyltriethoxysilane, a fluoroalkyltrimethoxysilane, a chloroalkyltrimethoxysilane, and mixtures thereof.

9. The process as claimed in claim 3, 5, 6, 7 or 8, **characterized in that** the molar proportions of tetramethoxysilane/other organosilicon precursor are between 0.97/0.03 and 0.6/0.4, preferably between 0.9/0.1 and 0.7/0.3.

10. The process as claimed in any one of the preceding claims, **characterized in that** the polar organic solvent is methanol.

11. The process as claimed in any one of the preceding claims, **characterized in that** the molar proportions of polar organic solvent and of water are, respectively, between 4 and 10, preferably between 4 and 7 and more preferentially between 4 and 5.

12. A nanoporous polyalkoxysilane sol-gel matrix containing carmine indigo, obtained from tetramethoxysilane or from a mixture of tetramethoxysilane and of another organosilicon precursor chosen from phenyltrimethoxysilane, phenyltriethoxysilane, a fluoroalkyltrimethoxysilane, a fluoroalkyltriethoxysilane, a chloroalkylmethoxysilane, a chloroalkylethoxysilane, an aminopropyltriethoxysilane, and mixtures thereof, **characterized in that** it has a specific surface area of from $550 \pm 60$ m$^2$.g$^{-1}$ to $890 \pm 80$ m$^2$.g$^{-1}$, preferably from $650 \pm 60$ m$^2$.g$^{-1}$ to $890 \pm 80$ m$^2$.g$^{-1}$, and even more preferentially from $750 \pm 70$ m$^2$.g$^{-1}$ to $890 \pm 80$ m$^2$.g$^{-1}$, and a proportion of micropores of greater than 30%, preferably greater than 40% and even more preferentially greater than 60%, the specific surface area and the pore size distribution being determined by analysis of the liquid nitrogen adsorption-desorption isotherm with the DFT model, the term "DFT" meaning density functional theory.

13. The use of a nanoporous polyalkoxysilane sol-gel matrix containing carmine indigo as claimed in claim 12 for selectively trapping ozone present in the air.

14. The use as claimed in claim 13, **characterized in that** the matrix is used as an air filter for ozone abatement.

15. The use as claimed in claim 13, **characterized in that** the matrix is used in the detection of ozone present in the air.

16. An ozone abatement filter, **characterized in that** it comprises a nanoporous polyalkoxysilane sol-gel matrix containing carmine indigo as claimed in claim 12.

17. An ozone sensor, **characterized in that** it comprises a nanoporous polyalkoxysilane sol-gel matrix containing carmine indigo as claimed in claim 12.

(A)                                              (B)

FIG. 1

(A)

(B)

FIG. 2

FIG. 3

FIG. 4

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1394541 A2
- US 4859607 A

- WO 2008056513 A1

**Littérature non-brevet citée dans la description**

- **CLIFFORD WEISEL ; CHARLES J. WESCHLER ; KRIS MOHAN ; JOSE VALLARINO JOHN D. SPENGLER ; OZONE.** Ozone Byproducts in the Cabins of Commercial Aircraft. *Environ. Sci. Technol.*
- **P. HURÉ ; X. ROUSSELIN.** l'émission d'ozone par les photocopieurs et imprimantes laser. *INRS,* 1997
- **A. GINESTET ; D. PUGNET.** L'efficacité des filtres moléculaires pour la ventilation des bâtiments. *Centre technique des industries aérauliques et thermique*
- **TODOR BATAKLIEV ; VLADIMIR GEORGIEV ; METODY ANACHKOV ; SLAVCHO RAKOVSKY ; GENNADI E. ZAIKOV.** Ozone decomposition. *Interdisciplinary Toxicology,* 2014, vol. 7 (2), 47-59
- **J.P VIRICELLE ; A. PAULY ; L MAZET ; J.BRUNET ; M BOUVET ; C. VARENNE ; C.PIJOLAT.** Selectivity improvement of semi-conducting gas sensors by selective filter for atmospheric pollutants detection. *Materials Science and Enginnering C,* 2006, vol. 26, 186-195
- **M. ALEXY ; G. VOSS ; J. HEINZE.** Optochemical sensor for determining ozone based on novel soluble indigo dyes immobilised in a highly permeable polymeric film. *Analytical and Bioanalytical Chemistry,* 2005, vol. 382, 1628-1641

- **M. YASUKO YAMADA.** Measurement of ambient ozone using newly developed porous glass sensor. *Sensors and Actuators B,* 2007, vol. 126, 485-491
- **G. KIRIAKIDIS ; J. KORTIDIS ; K. MOSCHOVIS ; D. DOVINOS.** On the Road to Inexpensive, sub-ppb, Room temperature Ozone Sensing. *Sensor Lett.,* 2008, vol. 6 (6), 812-816
- **ANNA C. FRANKLIN et al.** Ozone Measurement in South Carolina Using Passive Sampler. *Journal of the Air & Waste Measurement Association,* 2004, vol. 54, 1312-1320
- General Information on Vycor Glass No. 7930. Corning Glass Works
- **GAGO-FERRERO P ; DEMEESTERE K ; SILVIA DÍAZ-CRUZ M ; BARCELÓ D.** Ozonation and peroxone oxidation of benzophenone-3 in water: effect of operational parameters and identification of intermediate products. *Sci Total Environ,* 15 Janvier 2013, vol. 443, 209-17
- **FERNANDO J. BELTRAN.** Ozone reaction kinetics in water and waste water systems. Taylor & Francis, 2005